# EUROPEAN PATENT SPECIFICATION

(11) **EP 4 151 143 B1**
(45) Date of publication and mention of the grant of the patent: **11.09.2024**
(21) Application number: 21198267.3
(22) Date of filing: 22.09.2021
(51) Int. Cl.: A61B 5/00, A61B 5/107, G01B 5/00, G01B 11/24, G01B 21/04, G01S 17/89

(54) **OBJECT SCANNING DEVICE AND SYSTEM FOR SCANNING OBJECT, ESPECIALLY HUMAN FIGURE**
VORRICHTUNG UND SYSTEM ZUR ABTASTUNG VON OBJEKTEN, INSBESONDERE DER MENSCHLICHEN FIGUR
DISPOSITIF ET SYSTEME DE BALAYAGE D'OBJETS, NOTAMMENT DE LA FIGURE HUMAINE

(30) Priority: 20.09.2021 PL 43898721
(43) Date of publication of application: 22.03.2023
(73) Proprietor: Eduroco sp. z o.o, 90-562 Lodz (PL)
(72) Inventor: Trojnacki, Maciej, Warszawa (PL); Dabek, Przemyslaw, Lódz (PL); Pelka, Michal, Warszawa (PL); Jaroszek, Piotr, Pionki (PL); Mis , Piotr, Koszalin (PL); Brzostko, Barbara, Warszawa (PL)
(74) Representative: Wlasienko, Jozef

(56) References cited:
- CN-A- 111 700 332
- CN-U- 204 196 256

## Description

### FIELD OF THE INVENTION

Subject of the invention is a device and a system for scanning objects, especially a human figure, enabling quick, accurate and automated obtaining of three-dimensional models of object from multiple registered images and point clouds.

The solution pertains to generally automatic gathering of measurement data about the scanned objects of various sizes, fundamentally independent of its shape and construction, which allows for acquiring a 3D model of the object, especially the figure of a human, hence a complex measurement of shapes, for example, of a human body. Furthermore, the invention includes in its scope a system of 3D modelling of object, which the system for scanning objects is equipped with.

The present solution according to the invention in general pertains to the field of mechatronics and involves a connection of mechanical engineering, electrical engineering, computer engineering including information systems engineering, control engineering and robotics for designing and manufacturing a modern device and system for scanning objects, especially a human figure.

### BACKGROUND OF THE INVENTION

Development in the scanning field allows for the analysis of objects or the real-world environment in order to gather measurement data of its shape and often also its appearance. Then, the gathered data can be used for generation of a 3D model of an object.

3D scanning can also be used for creating a digital, three-dimensional model of the object, especially a human figure. The computer model can be used in a wide variety of applications like a three-dimensional object analysis, for example in the case of a human figure, to determine the dimensions of the body.

3D model of an object can be a virtual representation of a specific object being scanned, represented in the measuring data. For example, the three-dimensional contour, shape etc. of an object can be rendered to data representing a geometric form, which can be sent and/or saved in memory. 3D model of an object can be defined by a set of points in 3D space connected with different geometrical objects such as triangles, lines, curved surfaces etc. One of the potential ways of generating a 3D model of an object is 3D scanning of an object, which may involve collecting multiple scans of an object at different angles, i.e. pictures or point clouds.

In case of scanning of an object, especially a human figure, usually the user stands in a fixed position and then the system creates body contour maps based on depth sensors, projectors and visible light cameras or lasers. These systems then use the body contour maps to extract the measurements of the user. Determining the dimensions of the scanned object, especially the human figure, includes building a model of the scanned object using certain approach to generation of a three-dimensional model of the user's body, i.e. determining its anthropometric dimensional parameters.

In light of this, an important issue affecting the scanned object, especially the human figure, is the speed of collecting data about the object while maintaining high accuracy of its representation. The measurement data are photos obtained from digital cameras, which enables generation of a 3D model using the technique of digital photogrammetry or LIDAR sensors, which allows to obtain a 3D model in the form of the point cloud. 3D scanning can take place using a wide variety of scanning sensors and technologies suitable for this purpose.

### DESCRIPTION OF THE STATE OF THE ART

In the state of the art, there are many devices available for scanning objects which use various technologies, such as a laser technology or a structured light for creating a 3D file. Some scanning devices are better suited for certain applications, for example some are suitable for close range digitization of the small objects, while others better digitize large objects from medium and long distances. Scanning devices vary in size, speed, resolution and accuracy, moreover, they can use their own proprietary software and file types.

Most devices for scanning objects are based on a laser technology. This technology consists in laser measurements of the distance from the point with specified spatial coordinates to examined points of the scanned object and determination of their position in the adopted coordinate system. As a result of scanning the object a large number of measurement points is obtained - the so-called point cloud. Based on it, a three-dimensional map of the surface of the scanned object can be created.

In the state of the art, an international patent application No. WO/2018/070697A1 titled "*3D SCANNER*" is known, which describes a 3D scanner capable of acquiring scan data from all directions while simultaneously minimizing the number of 3D scanner sensors. The scanner is characterized in that it comprises: a support frame of a rotating frame assembly located at the upper end of the frame which surrounds the periphery of the scanned target object, horizontally with respect to the ground surface; a frame rotating assembly including a motor and a power transmission unit to supply the rotational power of the motor, mounted and fixed to the support frame of a rotating frame assembly; a horizontal frame section which is horizontal with respect to the ground surface, coupled to the power supply member; a vertical frame section formed by folding one side of the horizontal frame section; a rotating frame that rotates around the scanned target object as the power transmission unit rotates; multiple sensors of the 3D scanner mounted on the vertical section of the rotating frame for the purpose of scanning the target object; and a scanning control unit that controls the motor and multiple 3D scanner sensors to generate stereoscopic information about the scanned target object.

In addition, there is known in the state of the art the American patent No. US6734980 titled "*Body scanning equipment*", which presents a body scanning equipment that contains at least two sets of apparatus located at the front and at the rear respectively of a body to be scanned, and each set of apparatus has a scanning device, mirrors arranged on opposing sides of the scanning device for directing radiant energy to opposing sides of the body and a rotating mirror for directing radiant energy alternately between the scanning device and the first and the second mirrors. The scanning device is located in a booth for installation in a store.

There is also known the US patent application No. US20130296711 titled "*System and apparatus for automated total body imaging*", which shows a station / booth for automated full-body imaging that has a small footprint and enables quick, efficient, effective and consistent capture of multiple images of the user's or patient's body over time with minimal staff assistance.

In the state of the art, there is also known application No. CN 204 196 256 U entitled "*3D full-automatic scanning device*", which comprises a 3D scanner, an upper jacking frame and a lower jacking frame, wherein the upper jacking frame is fixedly connected with an indoor ceiling plate; the lower jacking frame is fixedly connected with the upper jacking frame; a mounting space is arranged between the upper jacking frame and the lower jacking frame; a closed annular sliding groove is formed in the upper surface of the lower jacking frame in a concave manner; a rotating rack is arranged inside the mounting space; the rotating rack is mounted on the upper jacking frame in a rotational manner; a pulley which is clamped into the closed annular sliding groove is arranged on the rotating rack; a rotation mechanism for controlling the sliding of the pulley is arranged on the rotating rack; a scanning space is arranged below the lower jacking rack; upright columns are vertically arranged on the rotating rack; a mounting base which can slide upwards and downwards and a lifting driving device for controlling the mounting base are arranged on the upright columns; the 3D scanner is mounted on the mounting base. Compared with the prior art, the 3D full-automatic scanning device can be used for scanning three-dimensional articles to be scanned or people who stand statically without rotation, so that people feel relatively comfortable in the scanning process, and the problem that the acquisition effect is poor or people feel uncomfortable when a conventional scanning acquisition mode is adopted is avoided.

Moreover, in the state of the art there are also known commercial solutions from, for example, TELMAT Industrie under the name "*TELMAT Industrie SYMCAD III*", or a solution produced by botspot AG, a German manufacturer, under the name of "*Doob Dooblicator*", which is a 3D body scanner. The solution in question has a scanning cabin, which is based on the photogrammetric 3D scanning technology, and the solution called "*botscan PRO S*" which allows to scan people and objects in order to obtain very precise, color 3D models which also uses the cabin.

The state of the art in the field of scanning devices for modelling an object, especially the figure of a human, shows that there are no intermediate and compromise solutions that allow obtaining a 3D model in a relatively short time, significantly shorter than in the case of using handheld scanners, and simultaneously at a relatively low price of the device compared to expensive, dedicated solutions characterized by high speed of operation and involving a large number of sensors.

The above-described solutions from the state of the art and more broadly solutions for scanning objects, which is not insignificant, obtain not very accurate measurements of the scanned object when trying to significantly shorten the scanning time, especially in the case of too unstructured or too complex objects, such as e.g., a human figure. Obtaining appropriately better measurement data requires extending the process of scanning the object significantly, which is important especially when scanning the human figure, where this time should, as a rule, be as short as possible due to the discomfort associated with the need to remain in a stationary pose for longer periods of time, which is particularly problematic for young children.

The vast majority of scanners available on the market has the form of a stand with the proper scanning device attached to it. Like the handheld scanners, they do not allow for the automation of the process of scanning an object, especially a human figure.

Therefore, in the state of the art there is no complementary device and system for scanning objects that would enable the automatic collection of measurement data of the scanned object, especially the human figure, while ensuring the appropriate speed of its scanning and maintaining the appropriate accuracy of this scanning, and thus collecting measurement data on the scanned object with appropriate quality, at the same time combining the possibility of scanning objects of various sizes, basically regardless of their shape and structure.

It should be also emphasized that the claimed solution according to the invention, in relation to the state of the art, is a more universal solution, easy to use by people with physical disabilities, while maintaining a relatively low price compared to solutions available on the market.

### SUMMARY OF THE INVENTION

The subject of the present invention is to develop a completely new solution in the form of a device and system for scanning objects, especially the figure of a human, which as a result should guarantee appropriate accuracy and repeatability of scanning of an object, especially a human figure, with a relatively shortened time of this scanning.

The present solution according to the invention should also provide such features and functionalities as:
- automation of the object scanning process enabling continuous operation of the device for scanning objects while reducing the necessary service by staff, or even eliminating it;
- in the case of personal scanning, ensuring intimacy by means of an optional housing of the device;
- optional ventilation of the interior of the housing;
- optional lamps for disinfecting of the device and interior of the housing;
- measuring the height of the scanned object especially of a person;
- providing access only to authorized users;
- lighting up the scanned object;
- the ability to configure the parameters of the device for scanning objects by the user in terms of scanning time and the resulting accuracy and/or repeatability of the model;
- preview of the obtained model on the user mobile device;
- the ability to voice control the device for scanning objects and receive voice notifications about its work;
- remote configuration and diagnostics of the device for scanning objects;
- processing in a cloud computing to accelerate measurement data processing and obtain a 3D model faster.

In order to achieve the above goals, according to one aspect of the present invention, it provides a device for scanning objects including an optional housing integrated therewith. The subject device for scanning objects has at least one mast with sensor sets and a support element, wherein the support element comprising a rotating arm, mounting connector designed to mount the device to an external object, for example, to a frame or room ceiling, and a body, wherein the mast is connected to the rotating arm and connected to the body, on which the mounting connector is located, and having a control unit for controlling the device, wherein the mast is shaped such that it rotates with the rotating arm around the stationary mounting connector, wherein at least one mast comprises at least one mast upper arm and at least one mast lower arm articulated with each other by means of a lower yoke forming a tilting structure of the mast in such a way that the mast lower arm is tilted in direction to the rotating arm with respect to the mast upper arm and mast upper arm is tilted in direction away from the rotating arm with respect to the mast lower arm, wherein the mast upper arm in its upper part is detachably connected to the rotating arm by means of an upper yoke.

Preferably, the mentioned tilting structure of the mast allows the mast upper arm to be tilted in direction away from the rotating arm with respect to the mast lower arm within the range of at least from 0 degrees to +45 degrees, and the mast lower arm to be tilted in direction to the rotating arm with respect to the mast upper arm within the range of at least from -45 degrees to 0 degrees.

Preferably, the mentioned sensor set on the mast is mounted by means of a sensor mounting connector on a trolley, which, by means of a stepper motor, moves longitudinally in a linear manner, respectively, along the mast upper arm and the mast lower arm to obtain a given number of positions in relation to the scanned object.

Preferably, the sensor set mounted on the sensor mounting connector has the ability to adjust the tilt angle by means of a servo-drive, thus adjusting to the scanned object in the range of at least +/- 45 degrees.

Preferably, the sensor set includes sensors enabling to collect data concerning the scanned object, in particular LIDAR sensors and/or magnetic sensors and/or cameras and/or smartphones equipped with cameras.

Preferably, a distance sensor is mounted in the body, preferably an ultrasonic sensor, designed to determine the height of the scanned object.

Preferably along the mast upper arm and/or along the mast lower arm a LED bar adjacent directly to the mast arms is integrated for lighting up the scanned object, and/or along the mast upper arm and/or along the mast lower arm there is at least one set of LED lamps not adjacent to the mast arms, fixed to the mentioned arms by means of a lighting mounting connector.

Preferably, the mentioned device for scanning objects is encased in with a structure in the form of a frame consisting of profiles with connectors, wherein the frame has a housing composed of plates with housing connectors that form the walls of the housing, wherein one of the walls of the housing is equipped with a door or a curtain.

Preferably, on the frame preferably in its upper part and/or in its corners are mounted the lamps for lighting up the scanned object and/or the disinfecting lamps in the form of virucidal and bactericidal lamps designed to clean the air in the housing.

Preferably, the door is equipped with an electric lock, supervised by a frame secondary control subsystem, which makes it possible to restrict access to the interior of the housing to authorized persons only.

Preferably, the housing is equipped with fans that enable adequate thermal comfort and air exchange inside the housing.

According to a further aspect of the present invention, the present invention provides a system for scanning an object, characterized in that it includes the device for scanning objects. This device for scanning objects is equipped with the following subsystems: a control subsystem C, a power subsystem P, a communication subsystem W, a sensor subsystem S and an arm drive subsystem DA, wherein the control subsystem C interacts with the power subsystem P containing DC converters and an external AC adapter, wherein between these subsystems C and P there is an interaction with the above-mentioned subsystems: the communication subsystem W, the sensor subsystem S and the arm drive subsystem DA, wherein the mentioned device for scanning objects is connected via a communication subsystem W to a wide area network WAN through which it interacts with an information system COS, external information systems EXT and user mobile devices UMD and/or an alternative system.

Preferably, the mentioned device for scanning objects is additionally equipped with the following subsystems: a trolley motion subsystem DT, a head tilting subsystem DH and a lighting subsystem DL, and/or housing ventilation subsystem DV and/or door electric lock subsystem DE and those subsystems interact with the control subsystem C and the power subsystem P.

Preferably, the mentioned information system COS has a web application IA, a computing application CA, a database DB and a data storage AD.

Preferably, the external information systems EXT include, in particular, external payment processing systems and social media systems.

The envisaged solution according to the aspects proposed above provides an appropriate device and a system for scanning objects, especially a human figure, enabling quick, accurate and automated 3D modelling of an object on condition of appropriate collection of measurement data on the scanned object, using the designed elements and systems according to the invention.

The developed solution according to the invention also has the ability to remotely set the parameters of the used sensors, e.g., in the case of cameras: depth of field, exposure time and sensitivity, by creating a module for selecting parameters of the sensors used to scan the object. The solution also has the option of turning off selected modules involved in generating the 3D model, so that in the event that we are dealing with the scanner user, he/she will be able to see the initial scan result faster. The proposed solution according to the invention is also equipped with software for removing artifacts, i.e., side effects of the operation of the 3D model generation algorithms, which are not part of the scanned object, especially the figure of the scanned person.

Thus, the proposed solution according to the invention leads to the automatic achievement of an accurate and repeatable 3D modelling of an object, especially a human figure. Obtaining such functionality is not possible in the case of devices for scanning objects available on the market, especially handheld scanners, and it is necessary when using 3D modelling of an object in such areas as, for example, printing figures, monitoring the progress of training or therapy, or selection of clothing.

The undoubted advantage of the claimed solution is that the solution according to the invention provides the generation of a 3D model with a relatively low number of errors related to changing the position of the scanned object, in particular, it concerns the scanned figure of a human, with a relatively short scanning time compared to solutions from the state of the art. The present solution provides lighting of the scanned object and also allows to measure its height. Moreover, the solution ensures intimacy of the scanned person through the use of housing. In addition, the optional housing of the device can be equipped with a ventilation system and disinfection lamps to ensure, on the one hand, greater comfort for the scanned persons and, on the other hand, safety of staying in a closed room. The present disinfection lamps are in the form of so-called virucidal and bactericidal lamps using UV-C radiation for air purification. Additionally, only authorized persons may have access to the present device, which is ensured by logging in to the system by a user and, in the case of using housing of the interior, by limiting physical access to the interior of the housing by means of a door with an electric lock.

### SHORT DESCRIPTION OF THE DRAWINGS

The subject matter of the invention is illustrated in the example implementation with reference to the attached drawings in which:
- **FIG. 1**: shows in various orthographic projections in top, front, side, rear and bottom views the device for scanning objects, in particular the human figure, according to the invention;
- **FIG. 2A - 2B**: shows in various orthogonal projections in top, front, and side views the device for scanning objects, in particular the figure of a human, according to the invention, in the example of implementation respectively with a frame in **FIG. 2A** and with the housing in **FIG. 2B****;**
- **FIG. 3A** - **3B**: shows in the form of an axonometric projection the device for scanning objects, in particular the figure of a human, according to the invention, in the example of implementation respectively with the frame in **FIG. 3A** and with the housing in **FIG 3B**;
- **FIG. 4**: shows in the form of an axonometric projection the device for scanning objects, in particular the figure of a human, according to the invention;
- **FIG. 5A - 5B**: shows the components of the mast upper arm and the mast lower arm of the device for scanning objects, respectively, in the example of implementation according to the invention;
- **FIG. 6A - 6B**: shows a support element of the device for scanning objects, in the example of implementation according to the invention;
- **FIG. 7**: shows the angular ranges for the mast upper arm and the mast lower arm of the device for scanning objects, in the example of implementation according to the invention;
- **FIG. 8**: shows a block diagram of a system for scanning objects according to the invention with the device for scanning objects according to the invention, in the example of implementation with a frame and with housing.

### DETAILED DESCRIPTION OF THE INVENTION

Below, the subject matter of the present invention is described in detail with reference to the attached Figures and examples of implementation. The present invention is not limited to the specific examples of implementation described herein.

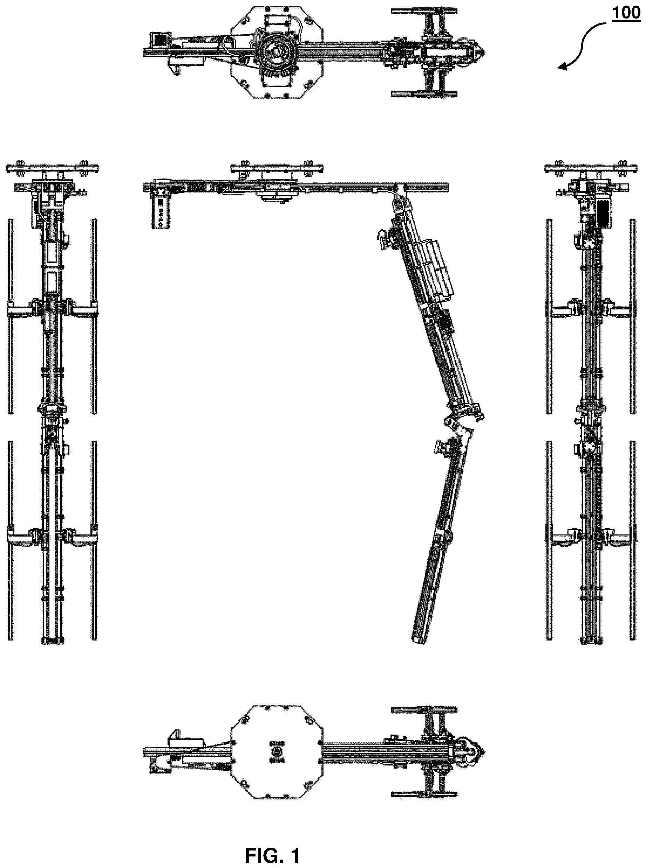

In the presented example of implementation in **FIG. 1** is illustrated in different orthographic projections in top, front, side, rear and bottom views the device 100 for scanning objects. The subject device 100 for scanning objects is designed to scan objects of various sizes, basically regardless of their shape and structure, and is also designed to automatically collect measurement data on the scanned object, especially the human figure.

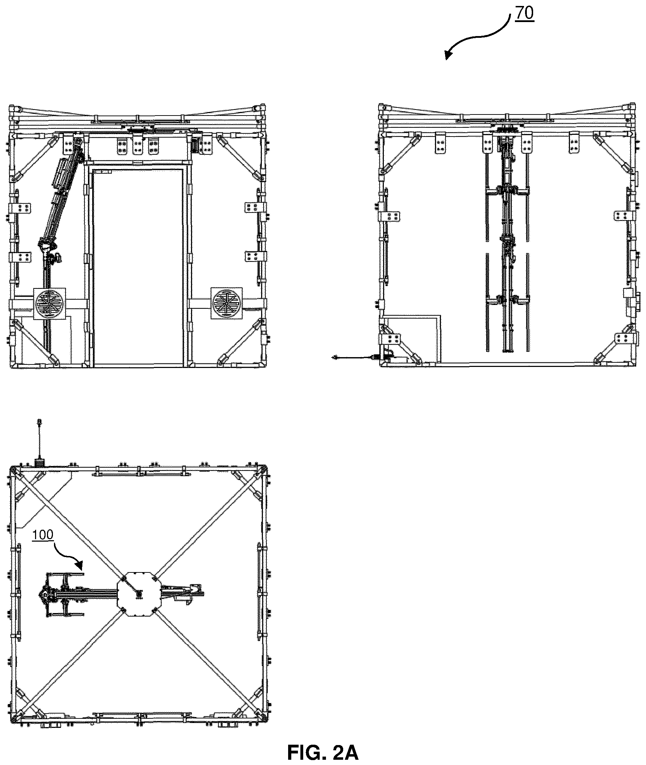

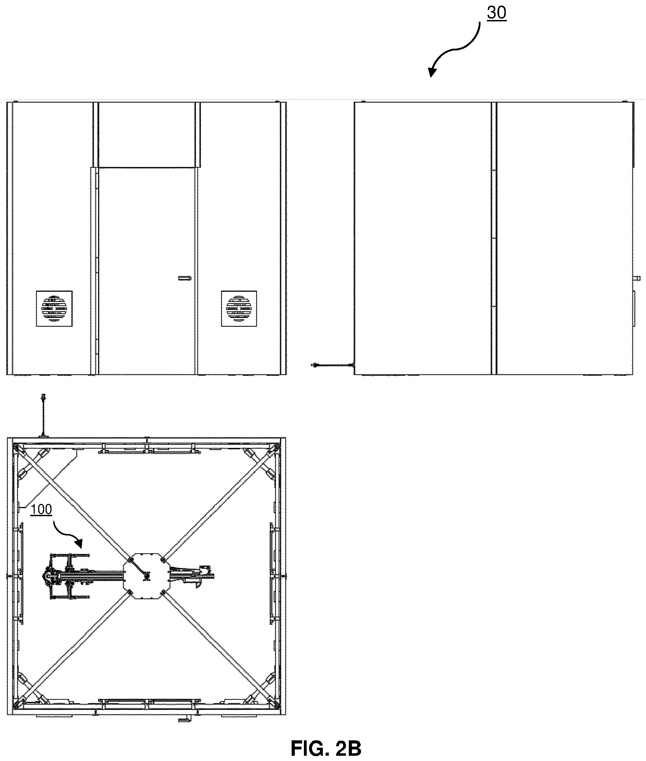

In turn, in the example of implementation **FIG. 2A** and **FIG 2B** illustrate in various orthogonal projections in top, front, and side views the device 100 for scanning objects according to the invention together with a structure integrated with it in the form of a frame 70 enclosed by the housing 30.

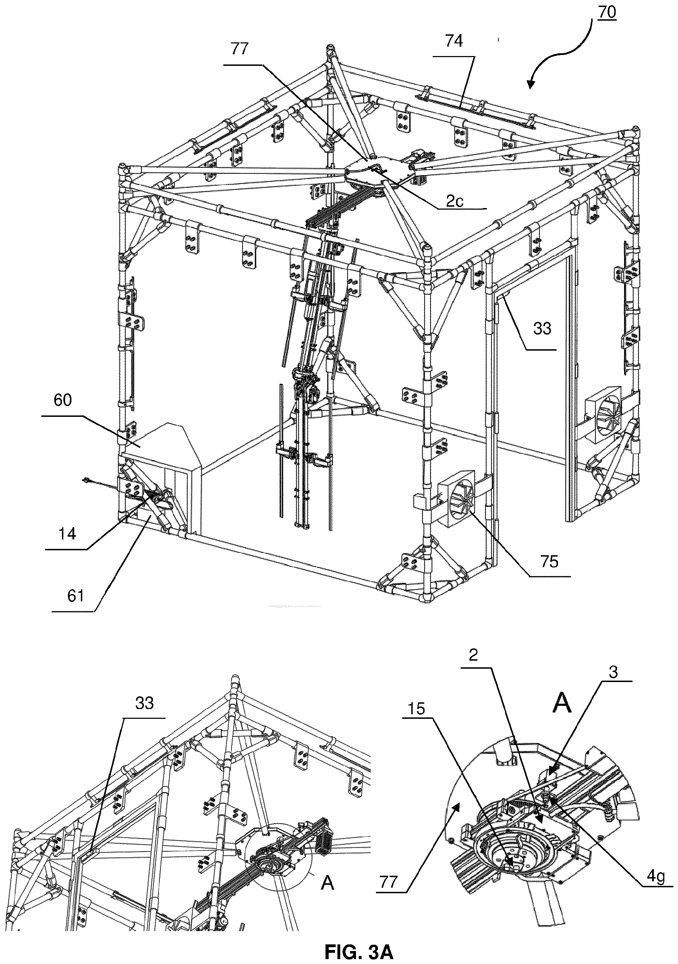

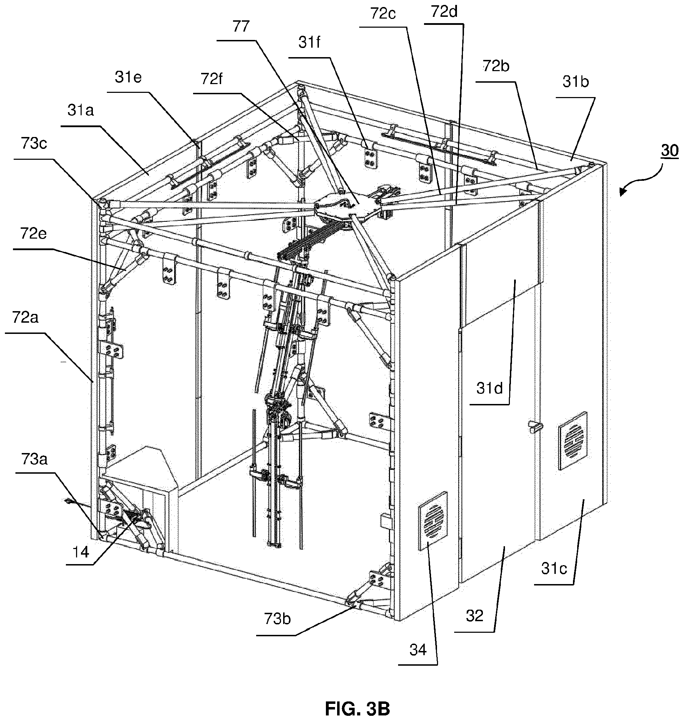

This structure is shown in more detail in **FIG. 3A** and **FIG. 3B** in the form of an axonometric projection, where it is illustrated with the device 100 for scanning objects according to the invention. The device 100 for scanning objects shown there is enclosed in the aforementioned structure in the form of a frame 70 consisting of profiles 72a-f with connectors 73a-c. The frame 70 in the example of implementation has a housing 30 composed of plates 31a-d with housing connectors 31e that form the walls of the housing 30. The mentioned frame 70 essentially consists of profiles, in particular, vertical 72a, horizontal 72b, diagonal 72c-d, stiffening 72e-f ones and connectors such as 73a-c connecting them. In one corner area of the frame 70 there is also a corner housing 60 in which an AC adapter 14 and a frame secondary control subsystem 61 are located.

In the example of implementation, the housing 30 comprises plates 31a-d, which are walls, housing connectors 31e, housing mountings 31f, door 32, electric lock 33 and fan covers 34. For a better visibility of the interior of the housing, in **FIG. 3B** are hidden on one side of the housing the plates 31b and the housing connector 31e. The housing 30 allows for providing the intimacy of the scanned person. In another variant of implementation, the housing may be additionally equipped with a ceiling or a floor. There is also possible a version of the solution, in which access to the interior of the housing is not authorized and there is a curtain instead of a door, or there is a door without an electric lock.

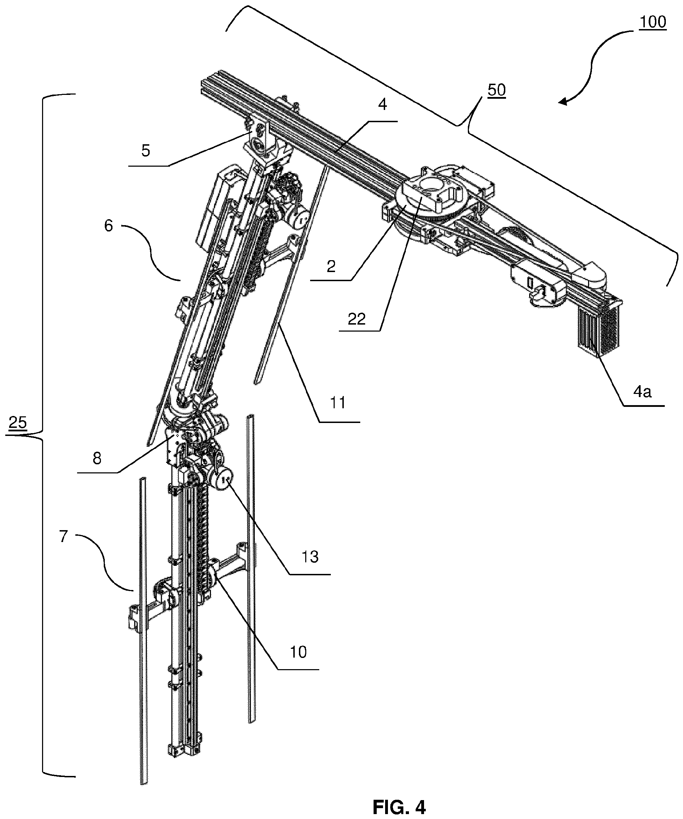

The stability of the presented structure is ensured by the mentioned frame 70, in the upper central part of which the device 100 for scanning objects is mounted. The mounting of the device 100 for scanning objects to the central part of the frame 70 is via a mounting connector 22 (see **FIG. 4**) designed to enable fastening to an external object, in this case, to the upper surface of e.g., frame 70 as in the present example of implementation. The mentioned mounting connector 22 in the example of implementation is fastened to the mounting plate 77 with screws (see **FIG. 3A** view A). The mounting plate 77 is part of the frame 70. In another variant of implementation, it is also possible to attach the mounting connector 22, e.g., to the ceiling of the room where the device 100 for scanning objects will be installed, but then an additional adapter is required that is attached directly to the ceiling from the bottom, since the mounting connector 22 is screwed with screws from the top.

Optional lamps 74 and fans 75, which are mounted only in the version of solution with housing 30, can also be mounted to the frame 70, as in the example of implementation. The mentioned lamps 74 in the example of implementation according to the invention are mounted in the upper part and in the corners of the housing 30. The mentioned lamps 74 play a dual role. On the one hand, they are designed to light up the scanned object, and on the other hand, they are designed to disinfec as virucidal and bactericidal lamps using UV-C radiation for cleaning the air in the housing 30.

In turn, **FIG. 4** illustrates in the form of an axonometric projection the structure of the device 100 for scanning objects, in particular the figure of a human, according to the invention. The present device 100 for scanning objects in this example of implementation has a single mast 25 and the support element 50 connected with it, situated above the mast 25, wherein the mast 25 is situated substantially vertically to the support element 50 under which the object to be scanned is placed. The support element 50 comprises a rotating arm 4, a mounting connector 22 intended to attach the device (100) to e.g., a frame 70 as described above, and a body 2, wherein the mast 25 is connected to a rotating arm 4 situated horizontally with respect to the mast 25 and connected to the body 2, on which the mounting connector 22 is located. The mast 25 according to the example of implementation is shaped such that it rotates with the rotating arm 4 around the stationary mounting connector 22. In the presented example of implementation, the mentioned mast 25 comprises the mast lower arm 7 and the mast upper arm 6 articulated with each other by means of an lower yoke 8 forming a tilting structure of the mast 25. In turn, the mast upper arm 6 in its upper part is detachably connected to the rotating arm 4 by means of an upper yoke 5.

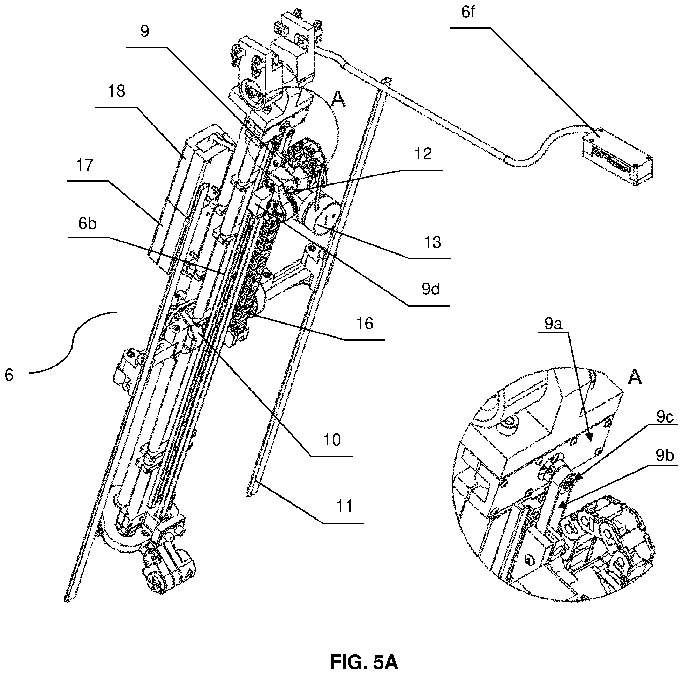

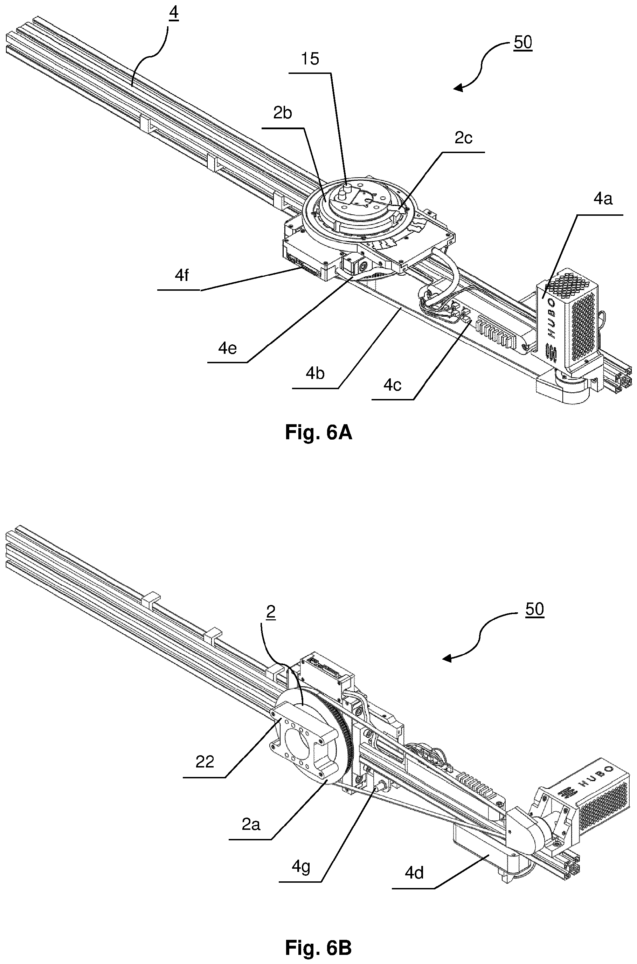

For controlling the device 100 for scanning objects is responsible the control unit consisting of the primary control subsystem 17 (see **FIG. 5A**) dedicated to controlling the operation of the present device 100 for scanning objects, including controlling the sensor set 13 (including triggering photos or scans), the secondary control subsystem 18 (see **FIG. 5A**) and the frame secondary control subsystem 61 (see **FIG. 3A**). The secondary control subsystem dedicated to controlling the drive assembly 4a and LED lamps 11, to operating the distance sensor 15, and to controlling the movement of two sensor sets 13 arranged in the example of implementation on the mast 25, one set on the upper arm 6 and the other set on the lower arm 7, respectively. In turn, for implementation of drive control of the rotating arm 4 is responsible the arm drive controller 4d illustrated in **FIG. 6B**. In turn, the frame secondary control subsystem 61 is responsible for controlling the equipment of the frame 70, i.e., lamps 74, fans 75 and electric lock 33.

In the example of implementation, a distance sensor 15 measuring the distance from the upper part of the scanned object is also mounted in the body 2 in its lower part. The present distance sensor 15 in the presented example of implementation is an ultrasonic sensor. It is shown in more detail in **FIG 3A** view A and **FIG. 6A**.

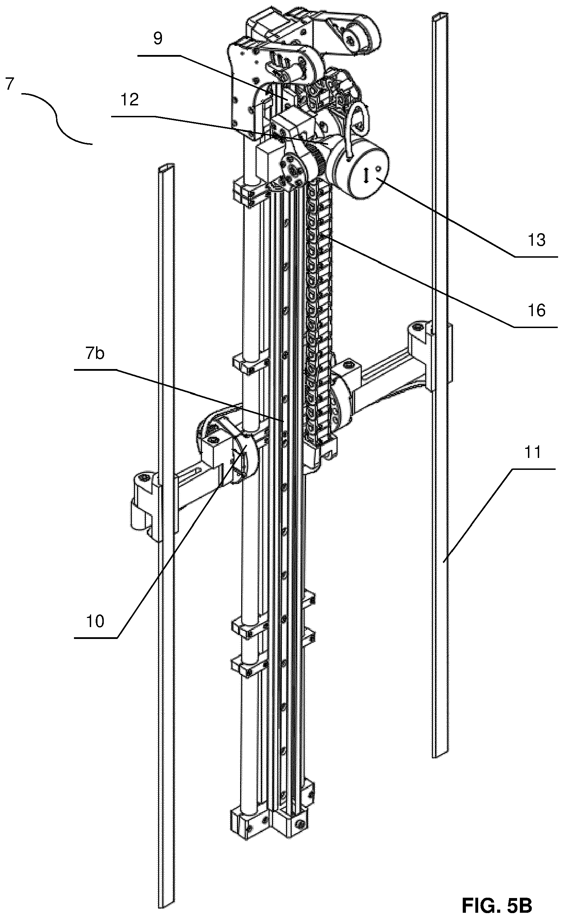

In **FIG. 5A** and **FIG. 5B** there are shown the components of the upper arm 6 and the lower arm 7 of the device 100 for scanning objects, respectively. In this example of implementation, the mast upper arm 6 and the mast lower arm 7 include one sensor set 13 mounted to the trolley 9 via a sensor mounting connector 12. The trolleys 9 automatically move, one on the trolley guide rail 6b and the other on the trolley guide rail 7b, respectively. The trolleys 9 are moved longitudinally in a linear manner along the mast upper arm 6 and the mast lower arm 7, respectively, to obtain a desired number of positions of the sensor set 13 with respect to the scanned object. The present trolley 9 is put in motion by the stepper motor 9a and via the drive transmission unit which includes a toothed belt 9b with a gear 9c which is illustrated in more detail in an enlarged manner in **FIG 5A** in view A for the upper arm 6 - an analogous solution was used for the lower arm 7. For the movement of the trolley 9 is responsible the secondary control subsystem 18 situated on the mast upper arm 6. Importantly for the present trolley 9, it is integrated with the cable carrier 16. The cable carrier 16 ensures that the cables are secured during the movement of the mentioned trolley 9, on the trolley guide rail 6b of the mast upper arm 6 and the trolley guide rail 7b of the mast lower arm 7, respectively.

In another variant of implementation, the range of movement of the trolley 9 on the mast upper arm 6 and the mast lower arm 7, respectively, and the tilt of the sensor set 13 are predetermined for the maximum assumed height of the scanned object, in particular the human figure. In another variant of implementation, the range of movement of the trolley 9 and the tilt of the sensor set 13 by means of the servo-drive 9d are automatically adjusted to the height of the scanned object, especially the human figure which, in the example of implementation, is achieved prior to starting the proper scan by performing measurement of the distance of the upper part of the scanned object from the distance sensor 15. The correct height of the object is obtained by subtracting the distance obtained by means of the distance sensor 15 from the known height at which the distance sensor 15 is located. In an alternative variant of implementation, in the case of the scanned object, which is the scanned person, one may enter one's own height in the mobile application. It is also worth emphasizing here that the scanning range of an object can also be narrower, i.e., it may cover a part of the scanned object, especially of the human figure. Thus, it can be assumed that the scanning range can be adjusted automatically to the scanned object, e.g., human height for full body scanning, or it can be limited to a user defined range.

In another variant of implementation, the mast upper arm 6 and the mast lower arm 7 each comprise two sensor sets 13 permanently mounted on the mast lower arm 7 and the mast upper arm 6, respectively. In this case, it is only possible to manually change the position of the mentioned sensor set 13 on the mast lower arm 7 and the mast upper arm 6, respectively.

In the realized example of implementation, the sensor set 13 mounted on the sensor mounting connector 12 has the ability to adjust the angle of tilt by means of a servo-drive 9d, thus adjusting to the scanned object in the range of +/- 45 degrees. The sensor set 13 consists of sensors that allow for collection of data about the scanned object and they may be magnetic sensors and/or cameras and/or smartphones equipped with cameras, as well as LIDAR sensors. In the realized example of implementation, the sensor sets 13 are LIDAR sensors.

In a more preferable variant of implementation according to the invention, along the mast lower arm 7 and along the mast upper arm 6 the lighting in the form of a LED bar is integrated for lighting up the scanned object. In another variant of implementation, the lighting of the scanned object is a set of LED lamps 11, which is located along the mast lower arm 7 and along the mast upper arm 6 in a way not adjacent to these arms, attached to them by means of a lighting mounting connector 10. Additionally, lamps 74 may be positioned on the frame 70. The purpose of the optional LED lamps 11 of the device 100 for scanning objects and lamps 74 of the frame is to ensure the appropriate lighting of the scanned object, when external lighting is not sufficient. The tilt and change of position of the LED lamps 11 are controlled via the lighting mounting connector 10.

In turn, in **FIG. 6A-6B** there is shown a support element 50 of the device 100 for scanning objects in the example of implementation according to the invention. The present support element 50 includes the following components: rotating arm 4 with a body 2. The mentioned rotating arm 4 is set in motion by means of a drive assembly 4a mounted on this arm and via a toothed belt 4b that cooperates with a gear 2a fixed to the bushing 2b.

Power for the device 100 for scanning objects in the example of implementation is supplied by an AC adapter 14 (see **FIG. 3A-3B**), designed to power all subsystems of the device with a voltage of 230 Vac / 24 Vdc. Power is transferred via the cable 2c shown in **FIG. 3A**, mounted on frame 70, to the rotary joint connector shown in **FIG. 6A** fixed to the bushing 2b, and then to the power distribution board 4c. The rotary joint connector (the detailed construction of which is not shown in figures) enables unlimited rotation of the rotating arm 4 together with the mast 25 in relation to the stationary mounting connector 22 and the body 2. According to **FIG. 6A** and **FIG. 6B**, the power distribution board 4c is connected to the arm drive controller 4d responsible for movement of rotating arm 4. Along the rotating arm 4, the cables are routed from the power distribution board 4c and the arm drive controller 4d in the form of a wire harness 4e. The wire harness 4e ends with a wire connector 4f which is connected to a wire connector 6f (shown in **FIG. 5A**) to provide power to the subsystems located on the mast arms 6 and 7 and communication with them.

A magnet 3 is attached to the lower part of the mounting plate 77 (visible in **FIG. 3A** view A), which cooperates with a magnetic field sensor 4g thus enabling the determination of the zero angular position of the rotating arm 4.

The above-described device 100 for scanning objects further has the ability to configure the working space of the present device to an assumed maximum size of the object to be scanned. In the realized example of implementation, the maximum height of the scanned object is 220 cm, which is carried out by fixing the mast upper arm 6 and the mast lower arm 7 with the upper yoke 5 at a predetermined distance from the axis of rotation of the rotating arm 4, which is equal to 80 cm ±20 cm.

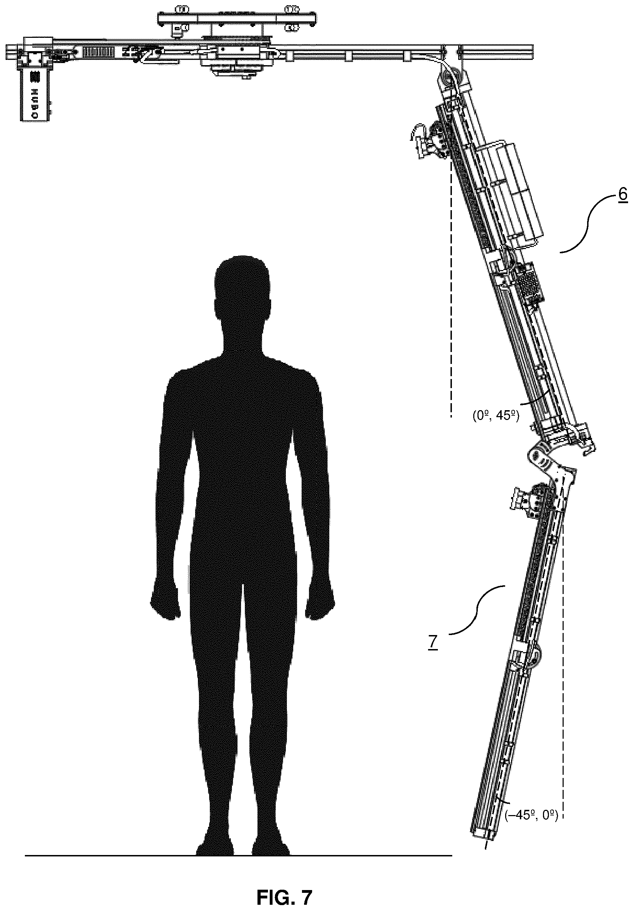

In turn, the setting of the desired joint angles in the upper yoke 5 ranges from 0 degrees to +45 degrees, and in the lower yoke 8 it ranges from 0 degrees to -45 degrees, which allows to obtain desired angles of tilt of the arms 6 and 7 of the mast, wherein the angle is measured from the vertical, as shown in **FIG. 7** illustrating the angular ranges for the above-mentioned arms 6 and 7 of the mast. A positive angle means the tilt of the mast upper arm 6 from the rotation axis outward of the device 100 for scanning objects. In turn, a negative angle means the tilt of the mast lower arm 7 from the rotation axis inward of the device 100 for scanning objects.

The above-mentioned lower yoke 8 and upper yoke 5 thus form the tilting structure of the mast 25, which enables tilting of the arms 6 and 7 of the mast and changing their distance from the axis of rotation to enable scanning of large objects with a complex geometric structure. Due to the articulated structure of the mast 25, it is possible to obtain a correspondingly better quality of the scanned object, because one looks at the scanned object slightly from above and from below, which in turn improves the quality of the obtained measurement data. The mentioned tilting structure allows the mast upper arm 6 to be tilted in the range from 0 degrees to +45 degrees, whilst the mast lower arm 7 in the range from - 45 degrees to 0 degrees, as mentioned above.

In the example of implementation according to the invention, the scanned object remains stationary and the device 100 for scanning objects by rotating the mast 25 scans the object from different sides, i.e., for the given poses of the sensor set 13. In one example of implementation, the scanning consists in collecting information about the scanned object in the form of point clouds, in which each point is obtained by measuring the distance from the LIDAR to the scanned object, at a given position of one of the LIDARs and for a given laser beam of this LIDAR, whilst the color of the point is obtained from the camera integrated with this LIDAR. According to one example of implementation, the desired poses of the sensor set 13 are obtained by rotation of the rotating arm 4 with the mast 25 and the possibility of tilting the sensors by means of the sensor mounting connector 12 and moving them in relation to the mast arms 6 and 7 by means of the trolleys 9, described above in the present description.

### Description of an example of operation of a scanning device

The device 100 for scanning objects is ready for operation once all subsystems have been powered on. In the example of implementation, prior to scanning an object, the mentioned device 100 performs a one-time homing process. This process consists in moving the rotating arm 4 together with the mast 25 from its current position to the zero position, which is always associated with rotating the mast 25 about the vertical axis until the zero position is detected by the magnetic field sensor 4g.

The process of scanning an object using the device 100 for scanning objects relies on gathering the data on the scanned object from the different positions of the sensor sets 13 around the scanned object, wherein the nature of the collected measurement data may be geometric, visual or other, depending on the type of used sensor sets 13, and it proceeds as follows:
- the mast 25 with the rotating arm 4 of the device 100 for scanning objects rotates in relation to the mounting connector 22 attached in the embodiment to the mounting plate 77 located on the frame 70, moving to the successive predetermined angular positions;
- the mentioned device 100 collects data at successive angular positions of the rotating arm 4, wherein in one variant of implementation, the trolleys 9, on which the sensor sets 13 are placed, can additionally move in each position, which allows for obtaining different positions of the sensor sets 13 with respect to the mast 25.

The above process is carried out until the entire object scanning process is completed, i.e., until the measurement data for all desired positions of the sensor sets 13 are acquired.

In view of the above, depending on the scanned object, i.e., its size, shape and structure, and therefore the complexity of its structure, the angular velocity is assumed to be in the range of 1-4 rpm. As a rule, the angular velocity can be adapted to the scanned object. It is possible to obtain a relatively high accuracy of the model by using a longer scanning time and collecting more measurement data, as well as obtaining a lower quality with a shorter scanning time and a smaller amount of measurement data. It is also possible to obtain a high-quality model in a short time thanks to the simultaneous use of more measuring sensors during scanning.

In example of implementation, when using the sensor set 13 in the form of LIDAR sensors, a complete scan of an object using present device 100 for scanning objects takes approximetely 30 seconds with two full rotations of the rotating arm 4 with the mast 25 and in the case of using 2 LIDARs, each of which during scanning reaches 2 extreme positions in relation to the arms 6 and 7 of the mast. On the other hand, for the configuration of the present device 100 for scanning objects in the form of 4 LIDARs mounted on the mast 25, the scanning takes approximetely 15 seconds with one complete rotation of the rotating arm 4 together with the mast 25.

Nevertheless, in the future new types of sensors may appear which in some application (e.g., medical or for creating animations in games) will be advantageous, but will require a lower or higher velocity of movement of the rotating arm 4 together with the mast 25 or the placement of more of these sensors on the mast 25 to obtain accurate measurement data.

In one example of implementation the sensor set 13 is a LIDAR located on a movable mast 25 with the structure defined above, wherein the mast 25 rotates around the scanned object. The key feature from the point of view of measurement accuracy when using this type of sensor set 13 is the number of scans. In the example of implementation 128 scans are obtained using the sensor set 13, wherein the scans are made around the scanned object in 32 positions by the rotation of the rotating arm 4 together with the mast 25, with simultaneous distribution of the scans vertically, in four positions of the sensor set 13 with respect to the mast 25. When two sensor sets 13 are used, the mentioned four their positions with respect to the mast 25 are obtained by moving the sensor sets 13 along the mast 25, wherein the rotating arm 4 performs two complete rotations during scanning. During the first rotation, the sensor sets 13 have the first extreme position with respect to the mast 25, and during the second rotation they have the second extreme position. Thus, during one full rotation of the rotating arm 4, the position of the sensor sets 13 with respect to the mast 25 is constant. In another example of implementation, four sensor sets 13 permanently mounted on the mast 25 can be used, which allows for obtaining the same number of scans, but for a single rotation of the rotating arm 4 together with the mast 25.

The present solution thus allows the device 100 for scanning objects to be configured for the purpose of determination of the model of the scanned object, i.e., adjusting it to the target application of this model, which is related to the collection of appropriate measurement data concerning the scanned object - the appropriate number of scans around the scanned object using LIDAR sensors. In another example of implementation, cameras can be used as sensors, which allows for the capturing of a sufficient number of photos of the scanned object, necessary to obtain its model. It is also possible to use several types of sensors simultaneously in one sensor set 13, e.g., LIDAR sensors and cameras.

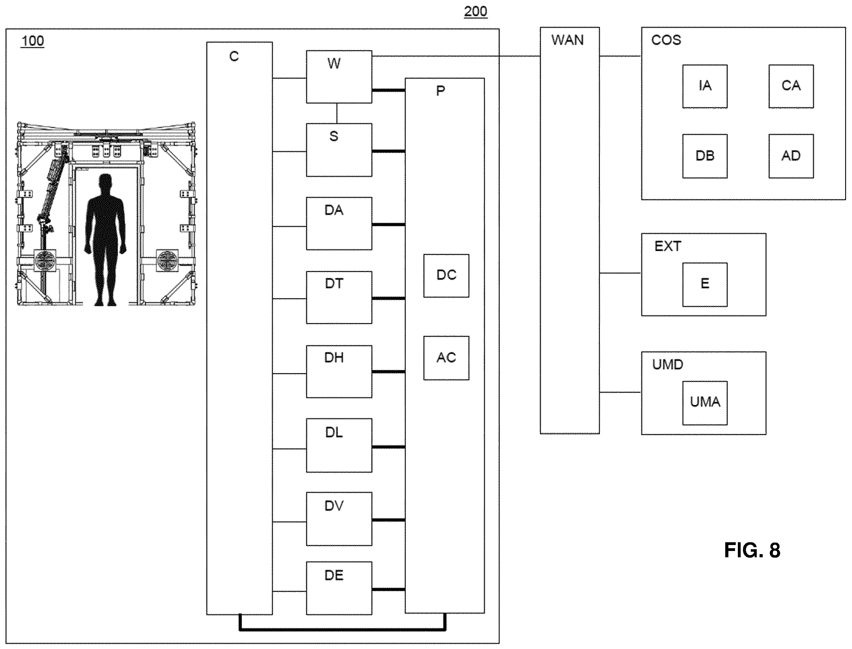

In **FIG. 8** a block diagram of the system 200 for scanning objects is shown in example of implementation of the present invention together with a device 100 for scanning objects integrated in the embodiment with the frame 70 with the housing 30. The mentioned system 200 for scanning objects interacts with the user mobile application UMA on the user mobile device UMD, with the information system COS and also optionally with external information systems EXT providing services, e.g., payments. System 200 for scanning objects including COS, UMD and EXT communicate with each other via wide area network WAN. Depending on the industry in which the scanning system will be used, in place of the user mobile application UMA on user mobile devices UMD, e.g., a desktop application for a PC type computer may be used.

The device 100 for scanning objects includes a power subsystem P, a sensor subsystem S, a control subsystem C, a communication subsystem W, in particular wireless communication, a housing ventilation subsystem DV, a door electric lock subsystem DE, an arm drive subsystem DA, a lighting subsystem DL, a trolley motion subsystem DT, and a head tilting subsystem DH. The trolley motion subsystem DT and the head tilting subsystem DH, which includes the sensor set 13, are optional elements and may be completely or partially omitted, e.g., in example of implementation of the device 100 for scanning objects, where the sensor set 13 is fixed directly on the mast 25 of the device 100 for scanning objects. Likewise, optional elements are the housing ventilation subsystem DV and the door electric lock subsystem DE, which can be omitted in the absence of housing of the device, i.e., if no doors and housing plates are attached to the frame.

The diagram shows the connections between the subsystems that are specifically responsible for power supply, communication with the WAN, and local communication within the device 100 for scanning objects.

The power subsystem P supplies all components and subsystems with the appropriate voltage, either directly or via DC / DC converters (DC). The source component of the power subsystem P may be a battery or an AC adapter (illustrated in **FIG. 3B** in the example of implementation as component 14) connected to the mains supply.

The sensor subsystem S comprises a sensor set 13, in which the sensors may in particular be cameras, cameras embedded in smartphones or LIDAR sensors. In the realized example of implementation LIDAR sensors are used as sensors. The number of sensors for each mast 25 of the device 100 for scanning objects may be between 1 and 12 depending on the type of sensor set 13 and the variant of the system 200 for scanning objects. The sensor subsystem also includes at least one height sensor of the object, which measures the distance from the sensor mounting location to the upper part of the object and, knowing the height of the sensor in relation to the ground, it determines the height of the scanned object. Measurement data from sensors are transferred to the control subsystem C via wired or wireless communication. Moreover, the selected measurement data is transferred using the communication subsystem W and via the wide area network WAN to the information system COS.

The control subsystem C includes computer resources responsible for data acquisition, coordinating the data collection process, and coordinating the operation of the entire system of the device 100 for scanning objects. In the example of implementation, it comprises a primary control subsystem 17, responsible for the operation of the entire system, including the operation of sensor sets 13, and a secondary control subsystem 18 and frame secondary control subsystem 61, which implements control commanded by the primary control subsystem 17. Depending on the sensors used in the sensor set 13, the primary control subsystem 17 may also be responsible for the computations related to the determination of the 3D model. The control subsystem C transfers by means of the primary control subsystem 17 the desired motion parameters to the secondary control subsystem 18. It, in turn, transfers the given motion parameters to the arm drive subsystems DA, which, in the example of implementation, includes an arm drive controller 4d, and optionally to the trolley motion subsystems DT and the head tilting subsystem DH, and obtains feedback from these subsystems. The control subsystem C also communicates the desired parameters of lighting to the lighting subsystem DL, which in the example of implementation includes LED lamps 11 and frame lamps 74, and also the desired parametrs of operation of the optional housing ventilation subsystem DV. In example of implementation, the LED lamps 11 and lamps 74 are directly controlled by the secondary control subsystem 18 and the frame secondary control subsystem 61, respectively. The control subsystem C also controls the operation of the optional door electric lock subsystem DE by means of the frame secondary control subsystem 61, thus allowing access of users to the inside of the housing of the device. The control subsystem C also cooperates with the information system COS, exchanging data with it and, through it, with user mobile applications UMA that operate on user mobile devices UMD or with other information systems, e.g., with a desktop application on a PC.

The communication subsystem W, in particular the wireless one, provides the control subsystem C with access to the wide area network WAN. This connection can be made through a connection to a local area network access point in the surroundings of the system 200, e.g., by means of a network card, in particular wireless one, either via a router or via a cellular modem. If the router is used, it can additionally create a local WiFi network of the scanner, which enables the operation of the sensor subsystem S, when the role of sensors is played by e.g., smartphones, and can additionally secure the information system against external access.

The arm drive subsystem DA consists of a drive assembly 4a (which includes a motor, a motor shaft rotation angle sensor or an absolute arm rotation sensor) and an optional sensor, in particular a magnetic field sensor 4g, for resetting the angular position of the arm of the device 100 for scanning objects in the absence of an absolute arm rotation angle sensor. The arm drive subsystem DA is responsible for the movement of the rotating arm 4 and is controlled by its arm drive controller 4d.

The lighting subsystem DL includes LED lamps 11 for the lower arm 7 and the upper arm 6 of the mast, and lamps 74 situated on the frame 70 of the device. The LED lamps are controlled by the secondary control subsystem 18, whilst the lamps 74 are controlled by the frame secondary control subsystem 61. The lamps may in particular be made of LED diodes emitting light of different wavelengths, including the infrared spectrum, depending on the requirements of the used sensors.

The trolley motion subsystem DT is optional and serves to move the sensor sets 13 along the mast lower arm 7 and mast upper arm 6. The trolley motion subsystem DT includes drive assemblies, which include: a stepper motor 9a and limit sensors. This subsystem is controlled by the secondary control subsystem 18.

The head tilting subsystem DH is optional and is used to tilt the sensor sets 13 by means of servo-drives 9d depending on their position along the length of the mast 25 and the tilt angle of the mast 25 itself. This subsystem is controlled by the secondary control subsystem 18.

The housing ventilation subsystem DV is optional and is used to ventilate the scanner surroundings when it is used to scan people and when enclosing the device with plates and doors. This subsystem is controlled by the frame secondary control subsystem 61.

The door electric lock subsystem DE is optional and is used to restrict access to the inside of the housing of the scanner, thus allowing entry for authorized persons only. This subsystem causes the door of the housing to be opened or closed. This subsystem is controlled by a frame secondary control subsystem 61. This subsystem does not exist in case of absence of housing of the device with walls and doors.

The integral part of the system 200 for scanning objects is the information system COS, which provides, for example, the ability to automatically obtain 3D models of scanned objects, the ability to support multiple users with ensuring access security and the ability to service a fleet of devices 100 for scanning objects. The information system COS consists of the web application IA module, computing application CA module, database DB module and data storage AD module. These modules operate as part of the IT infrastructure that enables immediate adjustment of the supply of services to the demand of users. The web application IA is the link between all major components of system 200 for scanning objects.

The database DB stores the data necessary for the operation of web applications (especially licenses and user accounts) and computing servers, as well as data related to the scan results and target 3D models.

The computing application CA is responsible for processing the data obtained during the scanning in order to obtain the final 3D models of the scanned objects.

The data storages AD are responsible for long-term storage of binary files necessary for creating models and of selected multimedia files.

Optional external information systems EXT, including external services E, e.g., payment systems, social media systems, external 3D model provider systems, can be connected to the information system COS.

System 200 for scanning objects is ready for operation when all subsystems are turned on. In the example of implementation, the device 100 for scanning objects before scanning an object performs the homing process once, which was already defined above. After collecting the measurement data, they are processed into a 3D model of the scanned object with an algorithm adequate to the type of measurement data (i.e., the type of used sensor set 13), e.g., photogrammetry techniques, SLAM, other, using the computing server CS. After completing the calculations, their result in the form of a model is sent to the user mobile application UMA installed on the user mobile device UMD or to another system, e.g., a desktop application installed on a PC, where the user can view the scanned object. If the industry of using the device 100 for scanning objects requires it, for the sake of security, the measurement data are deleted from the server at the moment the service is provided. Further, the user mobile device UMD is configured to manually control or voice control the device 100 for scanning objects and to receive optional voice notifications.

An integral part of the system 200 for scanning objects is the user mobile application UMA or an alternative information system, e.g., a desktop application that is used to operate the device 100 for scanning objects by the user, that allows for logging into the system, enabling/disabling the scanning and processing of data, presentation of 3D models and their use in various applications. The user mobile application UMA or alternative information system optionally supports the voice communication, i.e., recognizes the user's voice commands and generates voice notifications.

The above description of the examples of implementation is provided to enable any expert to make or use the present invention. Various modifications of the illustrated examples of implementation are also possible including all such changes, modifications and variations falling within the scope of the appended claims. Thus, the basic principles defined herein may be applied to other examples of implementation without departing from the scope of the invention. Thus, it is not the intention of the present invention to be limited to the examples of implementation disclosed herein, but to be in accordance with the broadest range consistent with the principles and novel features disclosed herein.

The present solution according to the invention thus offers by the above-mentioned technical means, as indicated in the Figures from **FIG. 1** to **FIG. 8**, a device and system for scanning objects, especially the human figure, using the designed elements and systems intended to automatically collect measurement data concerning the scanned object.

### APPLICATION OF THE INVENTION

The invention can be used in many industries where a 3D model of an object, especially a human figure, can be used. The potential application of the present solution covers, in particular, the clothing industry, including tailoring companies and clothing stores, where thanks to the scan, it will not be necessary to measure clothes, because artificial intelligence will itself assess the shape of the body and select the appropriate clothes.

This solution will also be used in the broadly understood fitness industry, where it will allow one to quickly and accurately measure the progress after exercise by measuring the increase in muscle mass or the progress of fat loss, as well as in the medical industry, especially in centers of therapy of posture defects and rehabilitation and in plastic surgery centers. The solution can also be successfully used in the video game industry, creating 3D models of scanned objects, including, in particular the human figure, e.g., of a player whose figure is then placed in post-production of the game.

### LIST OF REFERENCE DESIGNATIONS

| **100** | **device for scanning objects** | | |
|---|---|---|---|
| 2 | body | 11 | LED lamp |
| 2a | gear | 12 | sensor mounting connector |
| 2b | bushing | 13 | sensor set |
| 2c | cable | 14 | AC adapter |
| 3 | magnet | 15 | distance sensor |
| 4 | rotating arm | 16 | cable carrier |
| 4a | drive assembly | 17 | primary control subsystem |
| 4b | toothed belt | 18 | secondary control subsystem |
| 4c | power distribution board | 22 | mounting connector |
| 4d | arm drive controller | 25 | mast |
| 4e | wire harness | 30 | housing |
| 4f | wire connector | 31a-d | plate |
| 4g | magnetic field sensor | 31e | housing connector |
| 5 | upper yoke | 31f | housing mounting |
| 6 | upper arm | 32 | door |
| 6b | trolley guide rail | 33 | electric lock |
| 6f | wire connector | 34 | fan cover |
| 7 | lower arm | 50 | support element |
| 7b | trolley guide rail | 60 | corner housing |
| 8 | lower yoke | 61 | frame secondary control subsystem |
| 9 | trolley | 70 | frame |
| 9a | stepper motor | 72a-f | profiles |
| 9b | toothed belt | 73a-c | connectors |
| 9c | gear | 74 | lighting / disinfection lamp |
| 9d | servo-drive | 75 | fan |
| 10 | lighting mounting connector | 77 | mounting plate |

| **200** | **system for scanning objects** | | |
|---|---|---|---|
| **P** | **power subsystem** | **W** | communication subsystem |
| **AC** | **external AC** adapter | WAN | wide area network |
| DC | DC / DC converter | COS | information system |
| C | control subsystem | UMA | user mobile application |
| DA | arm drive subsystem | UMD | user mobile device |
| DT | trolley motion subsystem | EXT | external information systems |
| DH | head tilting subsystem | CA | computing application |
| DL | lighting subsystem | DB | database |
| DV | housing ventilation subsystem | AD | data storage |
| IA | web application | DE | door electric lock subsystem |
| S | sensor subsystem | E | external services |

## Claims

1. A device (100) for scanning objects having at least one mast (25) with sensor sets (13) and a support element (50), wherein the support element (50) comprises a rotating arm (4), a mounting connector (22) designed to mount the device (100) to an external object and a body (2), wherein the mast (25) is connected to the rotating arm (4) and connected to the body (2), on which the mounting connector (22) is located, and having a control unit for controlling the device (100), wherein the mast (25) is shaped such that it rotates with the rotating arm (4) around the stationary mounting connector (22), wherein at least one mast (25) comprises at least one mast upper arm (6) and at least one mast lower arm (7) articulated with each other by means of a lower yoke (8) forming a tilting structure of the mast (25) in such a way that the mast lower arm (7) is tilted in direction to the rotating arm (4) with respect to the mast upper arm (6) and mast upper arm (6) is tilted in direction away from the rotating arm (4) with respect to the mast lower arm (7), wherein the mast upper arm (6) in its upper part is detachably connected to the rotating arm (4) by means of an upper yoke (5).

2. The device (100) according to claim 1, **characterized in that** the tilting structure of the mast (25) allows the mast upper arm (6) to be tilted in direction away from the rotating arm (4) with respect to the mast lower arm (7) within the range of at least from 0 degrees to +45 degrees, and the mast lower arm (7) to be tilted in direction to the rotating arm (4) with respect to the mast upper arm (6) within the range of at least from -45 degrees to 0 degrees.

3. The device (100) according to claim 1, **characterized in that** the sensor set (13) on the mast (25) is mounted by means of a sensor mounting connector (12) on a trolley (9), which, by means of a stepper motor (9a), moves longitudinally in a linear manner, respectively, along the mast upper arm (6) and the mast lower arm (7) to obtain a given number of positions in relation to the scanned object.

4. The device (100) according to claim 3, **characterized in that** the sensor set (13) mounted on the sensor mounting connector (12) has the ability to adjust the tilt angle by means of a servo-drive (9d), thus adjusting to the scanned object in the range of at least +/- 45 degrees.

5. The device (100) according to claim 4, **characterized in that** the sensor set (13) includes sensors enabling to collect data concerning the scanned object, in particular LIDAR sensors and/or magnetic sensors and/or cameras and/or smartphones equipped with cameras.

6. The device (100) according to claim 1, **characterized in that** a distance sensor (15) is mounted in the body (2), preferably an ultrasonic sensor, designed to determine the height of the scanned object.

7. The device (100) according to claim 1, **characterized in that** along the mast upper arm (6) and/or along the mast lower arm (7) a LED bar adjacent directly to the mast arms, respectively, is integrated for lighting up the scanned object and/or along the mast upper arm (6) and/or along the mast lower arm (7) there is at least one set of LED lamps (11) not adjacent to the mast upper arm (6) and/or mast lower arm (7) respectively, fixed to the mentioned arms by means of a lighting mounting connector (10).

8. The device (100) according to claim 1, **characterized in that** is encased in with a structure in the form of a frame (70) consisting of profiles (72a-f) with connectors (73a-c), wherein the frame (70) has a housing (30) composed of plates (31a-d) with housing connectors (31e) that form the walls of the housing, wherein one of the walls of the housing is equipped with a door (32) or a curtain.

9. The device (100) according to claim 8, **characterized in that** on the frame (70) preferably in its upper part and/or in its corners are mounted lamps (74) for lighting up the scanned object and/or disinfecting lamps in the form of virucidal and bactericidal lamps designed to clean the air in the housing (30).

10. The device (100) according to claim 8 or 9, **characterized in that** the door (32) is equipped with an electric lock (33), supervised by a frame secondary control subsystem (61), which makes it possible to restrict access to the interior of the housing (30) to authorized persons only.

11. The device (100) according to claim 8, **characterized in that** the housing (30) is equipped with fans (75) that enable adequate thermal comfort and air exchange inside the housing (30).

12. A system (200) for scanning objects, **characterized in that** it comprises the device (100) for scanning objects according to any one of the preceding claims 1 to 11, wherein the device (100) is equipped with subsystems: a control subsystem (C), a power subsystem (P), a communication subsystem (W), a sensor subsystem (S) and an arm drive subsystem (DA), wherein the control subsystem (C) interacts with the power subsystem (P) containing converters (DC) and an external AC adapter (AC), wherein between the control subsystem (C) and the power subsystem (P), there is an interaction with the above-mentioned subsystems: the communication subsystem (W), the sensor subsystem (S) and the arm drive subsystem (DA), wherein the mentioned device (100) is connected via a communication subsystem (W) to a wide area network (WAN) through which it interacts with an information system (COS), external information systems (EXT) and user mobile devices (UMD) and/or an alternative system.

13. The system (200) according to claim 12, **characterized in that** device (100) is additionally equipped with the following subsystems: a trolley motion subsystem (DT), a head tilting subsystem (DH), a lighting subsystem (DL) and/or housing ventilation subsystem (DV) and/or door electric lock subsystem (DE), and those subsystems interact with the control subsystem (C) and the power subsystem (P).

14. The system (200) according to claim 12, **characterized in that** the information system (COS) has a web application (IA), a computing application (CA), a database (DB) and a data storage (AD).

15. The system (200) according to claim 12, **characterized in that** the external information systems (EXT) have, external payment processing systems and social media systems.

## Patentansprüche

1. Gerät (100) zum Scannen von Objekten aufweisend mindestens einen Mast (25) mit Sensorsätzen (13) und einem Trägerelement (50), wobei das Trägerelement (50) einen Schwenkarm (4), einen Montageanschluss (22), der zum Montieren des Gerätes (100) an einem externen Objekt ausgebildet ist, und einen Körper (2) umfasst, wobei der Mast (25) mit dem Schwenkarm (4) und mit dem Körper (2) verbunden ist, an dem sich der Montageanschluss (22) befindet, und eine Steuereinheit zum Steuern des Geräts (100) aufweist, wobei der Mast (25) so ausgebildet ist, dass er sich mit dem Schwenkarm (4) um den stationären Montageanschluss (22) dreht, wobei mindestens ein Mast (25) mindestens einen Mastoberarm (6) und mindestens einen Mastunterarm (7) umfasst, die über ein Unterjoch (8) gelenkig miteinander verbunden sind und eine Kippstruktur des Masts (25) bilden, derart dass der Mastunterarm (7) gegenüber dem Mastoberarm (6) in Richtung zum Schwenkarm (4) geneigt ist und der Mastoberarm (6) gegenüber dem Schwenkarm (4) weg am Mastunterarm (7) geneigt ist, wobei der Mastoberarm (6) in seinem oberen Teil über ein Oberjoch (5) mit dem Schwenkarm (4) lösbar verbunden ist.

2. Gerät (100) nach Anspruch 1, **dadurch gekennzeichnet, dass** die Kippstruktur des Mastes (25) ermöglicht, den Mastoberarms (6) in Bezug auf den Mastunterarm (7) in Richtung vom Schwenkarm (4) weg im Bereich von mindestens 0 Grad bis +45 Grad und den Mastunterarms (7) in Bezug auf den Mastoberarm (6) in Richtung vom Schwenkarm (4) im Bereich von mindestens -45 Grad bis 0 Grad zu neigen.

3. Gerät (100) nach Anspruch 1, **dadurch gekennzeichnet, dass** der Sensorsatz (13) am Mast (25) mittels eines Sensormontageverbinders (12) auf einem Trolley (9) montiert ist, der mittels eines Schrittmotors (9a) sich in Längsrichtung entlang des Mastoberarms (6) und des Mastunterarms (7) bewegt, um eine bestimmte Anzahl von Positionen in Bezug auf das gescannte Objekt zu erhalten.

4. Gerät (100) nach Anspruch 3, **dadurch gekennzeichnet, dass** der am Sensorbefestigungsstecker (12) montierte Sensorsatz (13) die Möglichkeit aufweist, den Neigungswinkel mittels eines Servoantriebs (9d) einzustellen und sich so im Bereich von mindestens ±45 Grad mit dem gescannten Objekt anzupassen.

5. Gerät (100) nach Anspruch 4, **dadurch gekennzeichnet, dass** der Sensorsatz (13) Sensoren, die Daten über das gescannte Objekt zu sammeln ermöglichen, darstellt, insbesondere LIDAR-Sensoren und/oder Magnetsensoren und/oder Kameras und/oder mit Kameras ausgestattete Smartphones.

6. Gerät (100) nach Anspruch 1, **dadurch gekennzeichnet, dass** im Körper (2) ein Anwesenheitssensor (15) montiert ist, vorzugsweise ein Ultraschallsensor, der dazu bestimmt ist, um die Höhe des gescannten Objekts zu bestimmen.

7. Gerät (100) nach Anspruch 1, **dadurch gekennzeichnet, dass** entlang des Mastoberarms (6) und/oder entlang des Mastunterarms (7) jeweils eine direkt an die Mastarme angrenzende LED-Leiste zur Beleuchtung des gescannten Objekts eingebaut ist und/oder entlang des Mastoberarms (6) und/oder entlang des Mastunterarms (7) mindestens ein Satz von LED-Lampen (11) angeordnet ist, die nicht an den Mastoberarm (6) und/oder Mastunterarm (7) angrenzen und mittels eines Beleuchtungsbefestigungssteckers (10) an den genannten Armen befestigt sind.

8. Gerät (100) nach Anspruch 1, **dadurch gekennzeichnet, dass** es mit einer Struktur in Form eines Rahmens (70) umhüllt ist, der aus Profilen (72a-f) mit Verbindern (73a-c) besteht, wobei der Rahmen (70) ein Gehäuse (30) aufweist, das aus Platten (31a-d) mit Gehäuseverbindern (31e) besteht, die die Wände des Gehäuses bilden, wobei eine der Wände des Gehäuses mit einer Tür (32) oder einem Vorhang ausgestattet ist.

9. Gerät (100) nach Anspruch 8, **dadurch gekennzeichnet, dass** am Rahmen (70) vorzugsweise in seinem oberen Teil und/oder in seinen Ecken Lampen (74) zum Beleuchten des gescannten Objekts und/oder Desinfektionslampen in Form von viruziden und bakteriziden Lampen montiert sind, die zum Reinigen der Luft im Gehäuse (30) bestimmt sind.

10. Gerät (100) nach Anspruch 8 oder 9, **dadurch gekennzeichnet, dass** die Tür (32) mit einem elektrischen Schloss (33) ausgestattet ist, das von einem sekundären Rahmenkontrollsubsystem (61) überwacht wird, wodurch der Zugang zum Inneren des Gehäuses (30) ausschließlich auf autorisierte Personen beschränkt werden kann.

11. Gerät (100) nach Anspruch 8, **dadurch gekennzeichnet, dass** das Gehäuse (30) mit Lüftern (75) ausgestattet ist, die einen ausreichenden thermischen Komfort und Luftaustausch im Inneren des Gehäuses (30) ermöglichen.

12. System (200) zum Scannen von Objekten, **dadurch gekennzeichnet, dass** es die Gerät (100) zum Scannen von Objekten gemäß einem der vorhergehenden Ansprüche 1 bis 11 enthält, wobei das Gerät (100) mit folgenden Subsystemen ausgestattet ist: einem Steuersubsystem (C), einem Energieversorgungssubsystem (P), einem Kommunikationssubsystem (W), einem Sensorsubsystem (S) und einem Armantriebssubsystem (DA), wobei das Steuersubsystem (C) mit dem Stromversorgungssubsystem (P) interagiert, ein Umformer (DC) und einen externen Akkumulator (AC) enthält, wobei zwischen dem Steuerssubsystem (C) und dem Energieversorgungssubsystem (P) eine Interaktion mit den oben genannten Subsystemen besteht: dem Kommunikationssubsystem (W), dem Sensorsubsystem (S) und dem Armantriebssubsystem (DA), wobei das genannte Gerät (100) über ein Kommunikationssubsystem (W) mit einem Weitverkehrsnetz (WAN) verbunden ist, und über das Informationssystem (COS), externen Informationssysteme (EXT) und mobilen Benutzergeräten (UMD) und/oder einem alternativen System interagiert.

13. Das System (200) gemäß Anspruch 12, **dadurch gekennzeichnet, dass** das Gerät (100) zusätzlich mit den folgenden Subsystemen ausgestattet ist: einem Trolley-Bewegungs-Subsystem (DT), einem Kopfneigungs-Subsystem (DH), einem Beleuchtungs-Subsystem (DL) und/oder einem Gehäusebelüftungssubsystem (DV) und/oder einem Türelektroschlosssubsystem (DE), und diese Subsysteme mit dem Steuersubsystem (C) und dem Energieversorgungssubsystem (P) interagieren.

14. Das System (200) gemäß Anspruch 12, **dadurch gekennzeichnet, dass** das Informationssystem (COS) eine Webapplication (IA), eine Computerapplikation (CA), eine Datenbank (DB) und einen Datenspeicher (AD) aufweist.

15. Das System (200) gemäß Anspruch 12, **dadurch gekennzeichnet, dass** die externen Informationssysteme (EXT), externe Zahlungsabwicklungssysteme und Social-Media-Systeme aufweisen.

## Revendications

1. Dispositif (100) pour scanner des objets comportant au moins un mât (25) avec des ensembles de capteurs (13) et un élément de support (50), dans lequel l'élément de support (50) comprend un bras rotatif (4), un connecteur de montage (22) conçu pour monter le dispositif (100) sur un objet externe et un corps (2), dans lequel le mât (25) est relié au bras rotatif (4) et relié au corps (2), sur lequel le connecteur de montage (22), et comportant une unité de commande pour contrôler le dispositif (100), dans lequel le mât (25) est formé de telle sorte qu'il tourne avec le bras rotatif (4) autour du connecteur de montage fixe (22), dans lequel au moins un mât (25) comprend au moins un bras supérieur du mât (6) et au moins un bras inférieur du mât (7) articulés entre eux au moyen d'un étrier inférieur (8) formant une structure inclinable du mât (25) de telle manière que le bras inférieur du mât (7) est incliné en direction du bras rotatif (4) par rapport au bras supérieur du mât (6) et que le bras supérieur du mât (6) est incliné en direction opposée au bras rotatif (4) par rapport au bras inférieur du mât (7), le bras supérieur du mât (6) dans sa partie supérieure étant relié de manière amovible au bras rotatif (4) au moyen d'un étrier supérieur (5).

2. Dispositif (100) selon la revendication 1, **caractérisé en ce que** la structure inclinable du mât (25) permet au bras supérieur du mât (6) d'être incliné en direction opposée au bras rotatif (4) par rapport au bras inférieur du mât (7) dans une plage d'au moins 0 degrés à +45 degrés, et au bras inférieur du mât (7) d'être incliné en direction du bras rotatif (4) par rapport au bras supérieur du mât (6) dans une plage d'au moins -45 degrés à 0 degrés.

3. Dispositif (100) selon la revendication 1, **caractérisé en ce qu**e l'ensemble capteur (13) sur le mât (25) est monté au moyen du connecteur de montage de capteur (12) sur un chariot (9), qui, au moyen d'un moteur pas à pas (9a), se déplace longitudinalement de manière linéaire, respectivement, le long du bras supérieur du mât (6) et du bras inférieur du mât (7) pour obtenir un nombre donné de positions par rapport à l'objet scanné.

4. Dispositif (100) selon la revendication 3, **caractérisé en ce que** l'ensemble de capteurs (13) monté sur le connecteur de montage de capteur (12) a la capacité de régler l'angle d'inclinaison au moyen d'un servomoteur (9d), ainsi s'adapter à l'objet numérisé dans une plage d'au moins ±45 degrés.

5. Dispositif (100) selon la revendication 4, **caractérisé en ce que** l'ensemble de capteurs (13) comprend des capteurs permettant de collecter des données concernant l'objet scanné, notamment des capteurs LIDAR et/ou des capteurs magnétiques et/ou des caméras et/ou des smartphones équipés avec des caméras.

6. Dispositif (100) selon la revendication 1, **caractérisé en ce qu**'un capteur de distance (15) est monté dans le corps (2), de préférence un capteur à ultrasons, conçu pour déterminer la hauteur de l'objet scanné.

7. Dispositif (100) selon la revendication 1, **caractérisé en ce que** le long du bras supérieur du mât (6) et/ou le long du bras inférieur du mât (7), une barre LED adjacente directement aux bras du mât, respectivement, est intégrée pour l'éclairage de l'objet scanné et/ou le long du bras supérieur du mât (6) et/ou le long du bras inférieur du mât (7), il y a au moins un ensemble de lampes LED (11) non adjacentes au bras supérieur du mât (6) et/ ou un bras inférieur du mât (7), respectivement, fixé aux bras mentionnés au moyen d'un connecteur de montage d'éclairage (10).

8. Dispositif (100) selon la revendication 1, **caractérisé en ce qu**'il est entouré d'une structure en forme de cadre (70) constitué de profilés (72a-f) avec des connecteurs (73a-c), dans lequel le cadre (70) comporte un boîtier (30) composé de plaques (31a-d) avec des connecteurs du boîtier (31e) qui forment les parois du boîtier, l'une des parois du boîtier étant équipée d'une porte (32) ou d'un rideau.

9. Dispositif (100) selon la revendication 8, **caractérisé en ce que** sur le cadre (70) de préférence dans sa partie supérieure et/ou dans ses coins sont montées des lampes (74) pour éclairer l'objet scanné et/ou des lampes de désinfection sous forme de lampes virucides et bactéricides destinées à assainir l'air du boîtier (30).

10. Dispositif (100) selon la revendication 8 ou 9, **caractérisé en ce que** la porte (32) est équipée d'une serrure électrique (33), supervisée par un sous-système de commande secondaire du cadre (61), qui permet de restreindre l'accès à l'intérieur du boîtier (30) aux personnes autorisées uniquement.

11. Dispositif (100) selon la revendication 8, **caractérisé en ce que** le boîtier (30) est équipé de ventilateurs (75) qui permettent un confort thermique et un échange d'air adéquats à l'intérieur du boîtier (30).

12. Système (200) pour scanner des objets, **caractérisé en ce qu**'il comprend le dispositif (100) pour scanner des objets selon l'une quelconque des revendications précédentes de 1 à 11, dans lequel le dispositif (100) est équipé de sous-systèmes suivants: un sous-système de contrôle (C), un sous-système de puissance (P), un sous-système de communication (W), un sous-système de capteur (S) et un sous-système d'entraînement de bras (DA), le sous-système de commande (C) interagissant avec le sous-système de puissance (P) contenant des convertisseurs (DC) et un adaptateur secteur externe (AC), dans lequel entre le sous-système de commande (C) et le sous-système de puissance (P), il y a une interaction avec les sous-systèmes mentionnés ci-dessus: le sous-système de communication (W), le sous-système de capteur (S) et le sous-système d'entraînement de bras (DA), le dispositif mentionné (100) étant connecté via un sous-système de communication (W) à un réseau étendu (WAN) à travers lequel il interagit avec un système d'information (COS), des systèmes d'information externes (EXT) et les appareils mobiles des utilisateurs (UMD) et/ou un système alternatif.

13. Système (200) selon la revendication 12, **caractérisé en ce que** le dispositif (100) est en outre équipé des sous-systèmes suivants: un sous-système de déplacement du chariot (DT), un sous-système d'inclinaison de la tête (DH), un sous-système d'éclairage (DL) et /ou un sous-système de ventilation de boîtier (DV) et/ou un sous-système de verrouillage électrique de porte (DE), et ces sous-systèmes interagissent avec le sous-système de commande (C) et le sous-système d'alimentation (P).

14. Système (200) selon la revendication 12, **caractérisé en ce que** le système d'information (COS) comporte une application web (IA), une application informatique (CA), une base de données (DB) et un stockage de données (AD).

15. Système (200) selon la revendication 12, **caractérisé en ce que** les systèmes d'information externes (EXT) disposent de systèmes externes de traitement des paiements et de systèmes de médias sociaux.
